# EUROPEAN PATENT APPLICATION

(11) **EP 4 835 079 A1**
(43) Date of publication of application: **04.11.2026**
(21) Application number: 24912886.9
(22) Date of filing: 25.12.2024
(51) Int. Cl.: C12N 15/12, C12N 5/10

(54) **NOVEL DOUBLE-STRANDED RNA BASED ON C3 RNA SEQUENCE AND USE THEREOF**

(30) Priority: 27.12.2023 JP 2023221596
(71) Applicant: Toagosei Co., Ltd., Minato-ku Tokyo 105-8419 (JP)
(72) Inventor: BAILEYKOBAYASHI, Nahoko, Tokyo 105-8419 (JP); YOSHIDA, Tetsuhiko, Tokyo 105-8419 (JP); BAILEY, Eric Nelson, Tsukuba-shi, Ibaraki 305-0032 (JP)
(74) Representative: Bals & Vogel Patentanwälte PartGmbB
(86) International application number: PCT/JP2024/045859
(87) International publication number: WO 2025/143000

(57) **Abstract**

A double-stranded RNA disclosed herein includes a first strand and a second strand that is complementary to the first strand. The first strand includes a main sequence including 19 or more and 23 or less bases, in which a base at a 5'-terminus is guanine (G) or cytosine (C), and an additional sequence including two or more and four or less bases added to a 3'-terminus of the main sequence. Here, the main sequence includes a part of a base sequence coding for a complement component C3, which is at least a part of a base sequence coding for a signal peptide region of the complement component C3.

## Description

### [Technical Field]

The present disclosure relates to a double-stranded RNA and a composition including the double-stranded RNA, and a method of using these. Specifically, the present disclosure relates to a double-stranded RNA used for suppressing or inhibiting the growth or metastasis of a tumor cell, and a composition including the double-stranded RNA. The present application claims priority on the basis of Japanese Patent Application No. 2023-221596 filed on December 27, 2023, and the entire content of this application is incorporated into the description of the present application by reference.

### [Background Art]

A complement system is a part of an innate immune system, protecting a body from infection with pathogens such as bacteria and virus. The complement system includes several tens of kinds of proteins that operate on each other. The proteins in the complement system are created in liver mainly and circulate in blood and an extracellular fluid. Most part of the complement system is normally inactive but is activated by the infection with a bacterial or viral pathogen, for example. There are three pathways for activating the complement and the operations occur in the separate ways: a classical pathway (a first pathway), a lectin pathway (a mannose binding lectin pathway), and an alternative pathway (a second pathway). The activation of the complement system causes, for example, elimination of pathogens by a membrane attack complex (MAC), migration of phagocytes and lymphocytes, and opsonization. It is suggested, however, excess activation or inappropriate regulation of the complement will be related to an autoimmune disease, an inflammatory disease, growth and metastasis of a tumor cell, or the like in the end.

The initial component of each pathway described above works locally; however, any pathway converges to the activation of a complement component C3 (hereinafter also called "C3" simply). In the activation of C3, C3 is decomposed into C3b and C3a. C3b is covalently bonded to a surface of a pathogen to attract a fragment of C2 and C4, a complement B factor, or the like, thereby forming a complex. The complex with C3b cleaves C5, which is a late component, into C5a and C5b. The cleaved C5b is bound with C6, 7, 8, and 9, thereby forming a MAC. C3a and C5a alone operate as a diffusible signal and attract phagocytes and lymphocytes to an infection site, thereby promoting an inflammatory reaction. C3 plays a main role in the complement activating pathway as described above; thus, a medical composition that inhibits the activation of C3 and the expression of C3 is disclosed. Japanese Translation of PCT International Application Publication No. 2004-520287 discloses an antibody medical composition targeting C3. In addition, Japanese Translation of PCT International Application Publication No. 2009-521234 discloses siRNA targeting C3.

### [Citation List]

### [Patent Literatures]

[Patent Document 1] Japanese Translation of PCT International Application Publication No. 2009-520287
[Patent Document 2] Japanese Translation of PCT International Application Publication No. 2009-521234

### [Summary of Invention]

### [Technical Problem]

The antibody medicine and the like cost high and it is difficult to keep the quality uniform. The nucleic acid medicine can be mass-produced by organic synthesis and the uniformity of the quality is easily managed. The present inventor has paid attention to nucleic acid medicine, particularly to a signal peptide region of C3 that plays a main role in a complement system.

It is a main object of the present disclosure to provide a technique that can suppress or inhibit the growth of cells related to the gene expression of the complement component C3.

### [Solution to Problem]

A double-stranded RNA disclosed herein includes a first strand and a second strand that is complementary to the first strand. The first strand includes a main sequence including 19 or more and 23 or less bases, in which a base at a 5'-terminus is guanine (G) or cytosine (C), and an additional sequence including two or more and four or less bases added to a 3'-terminus of the main sequence. Here, the main sequence is determined from the base sequence including a part of a base sequence coding for a complement component C3, which is at least a part of a base sequence coding for a signal peptide region of the complement component C3.

The aforementioned double-stranded RNA can function as at least a small interfering RNA (siRNA). That is to say, it is predicted that such a double-stranded RNA causes RNA interference (RNAi). Such an effect inhibits the growth of the cells related to the complement component C3 because the expression of at least the complement component is suppressed.

In the double-stranded RNA according to one aspect disclosed herein, the second strand includes a main sequence complementary to the first strand, and an additional sequence including two or more and four or less bases added to a 3'-terminus of the complementary main sequence. Such a double-stranded RNA can function suitably as the siRNA. Thus, the growth of the cell related to the complement component can be inhibited more surely.

In the double-stranded RNA according to one aspect disclosed herein, at least three bases among five bases on the 3'-terminus of the main sequence are adenine (A) and/or uracil (U). Thus, the expression of the complement component C3 can be suppressed more sufficiently; therefore, the growth of the cell related to the complement component is inhibited.

In the double-stranded RNA according to one aspect disclosed herein, the base sequence coding for the complement component C3 includes any of the following base sequences:
GCCTGCTGCTCCTGCTACT (SEQ ID No.: 1);
CCTGCTGCTCCTGCTACTA(SEQ ID No.: 2);
CTGCTGCTCCTGCTACTAA (SEQ ID No.: 3); and
CTCTGGGGAGTCCCATGTA (SEQ ID No.: 4).
Such a double-stranded RNA suppresses the expression of the complement component C3 more specifically. Thus, the growth of the cell in which the amount of expression of the complement component C3 has increased can be inhibited.

In the double-stranded RNA according to one aspect disclosed herein, a base sequence included in the additional sequence is thymine-thymine (TT). Thus, the stability of the double-stranded RNA can be improved.

According to the present disclosure, a composition that can inhibit the growth of at least one kind of cell is provided. One aspect of the composition disclosed herein includes a double-stranded RNA including a first strand and a second strand that is complementary to the first strand, in which the first strand includes a main sequence including 19 or more and 23 or less bases where a base at a 5'-terminus is guanine (G) or cytosine (C), and an additional sequence including two or more and four or less bases added to a 3'-terminus of the main sequence, and the main sequence is determined from a base sequence including a part of a base sequence coding for a complement component C3, which is at least a part of a base sequence coding for a signal peptide region of the complement component C3. In the case of being supplied to the cell, such a composition suppresses the expression of at least the complement component C3; therefore, the growth of the cell related to the complement component is inhibited.

In one aspect of the composition disclosed herein, the cell species whose growth is inhibited by the composition is a tumor cell. Thus, the growth of the cell can be inhibited more surely.

One aspect of the composition disclosed herein includes a peptide fragment with cell membrane permeability that can transfer a foreign substance from outside a cell to an inside of a cytoplasm through a cell membrane. Thus, the double-stranded RNA can be easily transferred to a target cell.

The present disclosure provides a method of inhibiting growth of at least one kind of cell. One aspect of the method disclosed herein includes: (1) a step of preparing the composition disclosed herein; and (2) a step of supplying the composition in vitro to a target cell. Thus, the growth of the cell related to the complement component C3 can be inhibited.

In one aspect of the method disclosed herein, a biological species of the cell is the same as a biological species including the complement component C3. Thus, the growth of the cell related to the complement component C3 can be inhibited more surely.

### [Brief Description of Drawings]

Fig. 1 is a schematic view showing an outline of a complement pathway.
Fig. 2 is a graph showing cell viability of neuroblastoma cells in Samples 1 to 5 and Comparative Example.
Fig. 3 is a graph showing the cell viability of the neuroblastoma in the different amounts of addition in Samples 1 to 4 and Comparative Example.
Fig. 4 is a graph showing the cell viability of breast cancer cells in Samples 1 to 4 and Comparative Example.
Fig. 5 is a graph showing the cell viability of lung cancer cells in Sample 2 and Comparative Example.

### [Description of Embodiments]

### <Term definition>

The art disclosed herein will be described below in detail. Matters that are other than matters particularly mentioned in this specification (for example, a structure of a double-stranded RNA) and that are necessary for the implementation of the present art (for example, general matters related to synthesis methods for polynucleotide, cell culture techniques, constructs containing peptides or nucleic acids as a main component, and the like) can be grasped as design matters of those skilled in the art based on the prior art in the fields such as cell engineering, physiology, medical science, pharmacology, organic chemistry, biochemistry, genetic engineering, protein technology, molecular biology, and genetics. The art disclosed herein can be carried out on the basis of the contents disclosed in this specification and common technical knowledge in the fields.

The term "polynucleotide" in this specification refers to a polymer in which a plurality of (two or more) nucleotides are bound through a phosphoric diester bond, and is not limited by the number of nucleotides. For example, one containing both a deoxyribonucleotide and a nucleotide as nucleotides is also included in "the polynucleotide" in this specification. In addition, the term "artificially designed polynucleotide" in this specification refers to a polynucleotide of which nucleotide chains (full length) are not present in nature on their own and which is artificially synthesized through chemical synthesis or biosynthesis (that is, production based on genetic engineering).

In this specification, one of "a first strand" and "a second strand" is a sense strand (or a code strand or a passenger strand) and the other is an antisense strand (or a template strand, a non-code strand, or a guide strand). That is to say, when the first strand is the sense strand, the second strand is the antisense strand. In addition, when the second strand is the sense strand, the first strand is the antisense strand. The first strand and the second strand may be complementary to each other either completely or at least partially. That is to say, it is only necessary that the first strand and the second strand are capable of hybridization under at least a physiological condition.

In this specification, the left side of a base sequence always indicates a 5'-terminus and the right side thereof always indicates a 3'-terminus unless notation of "5'" and "3'" is added. In addition, the term "amino acid residue" in this specification encompasses N-terminus amino acids and C-terminus amino acids of a peptide chain unless otherwise stated specifically. Moreover, the amino acid sequence in this specification always expresses the N-terminus on the left side and the C-terminus on the right side.

In this specification, the term "tumor" is broadly interpreted, and refers to general tumors (typically, malignant tumors) including carcinoma and sarcoma, and lesions of blood and hematopoietic tissues (such as leukemia and lymphoma). In addition, "the tumor cell" has the same meaning as "a cancer cell" and refers to a cell that forms such a tumor and has typically grown abnormally regardless of the peripheral normal tissue (so-called a cell that has become cancerous). Therefore, a cell that is categorized as the tumor cell (cancer cell), not the normal cell, will be called the tumor cell regardless of the origin or properties of the cell unless otherwise defined especially.

In this specification, a numerical range described as "A to B (here, A and B are arbitrary numbers)" means "A or more and B or less" and additionally encompasses the meanings of "more than A and less than B", "more than A and B or less", and "A or more and less than B".

### <Complement component>

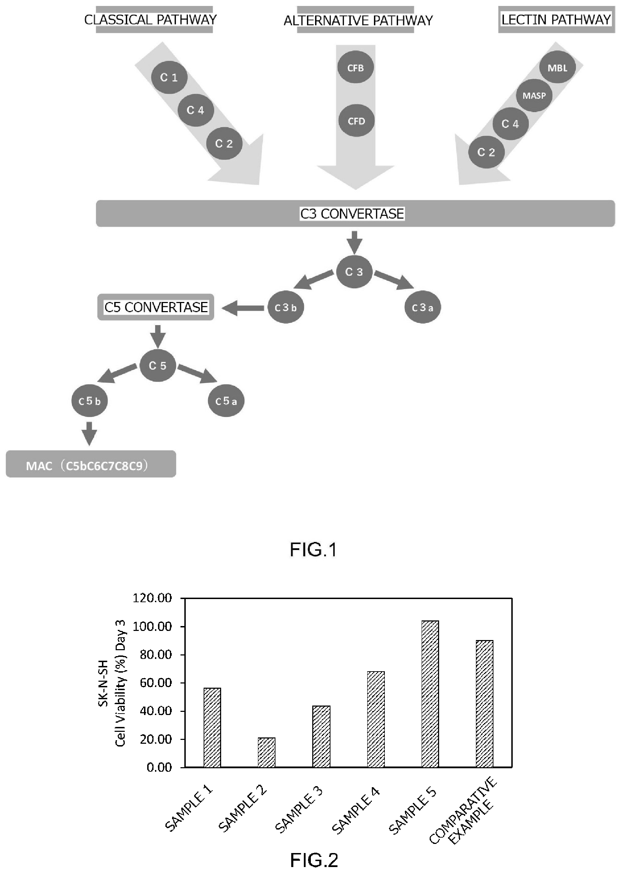

In this specification, the term "complement component" refers to a protein that is related to a complement pathway and its activation. Examples of the complement component include C1 (C1r, C1s, C1p), C2, C3, C4, C5, C6, C7, C8, C9, CFB, CFD, MBL, MASP, and the like. Fig. 1 is a schematic view showing an outline of complement activation. In a classical pathway, complement components C1, C2, C4, and the like are related mainly. The classical pathway is started when an antibody molecule of IgM or IgG is bound with a complement component C1q. C1r and C1s are activated and C2 and C4 are decomposed into C2a and C2b, and C4a and C4b, respectively. After that, C4b and C2a form a complex. The C4b-C2a complex works as a C3 convertase and decomposes C3 into C3a and C3b. An alternative pathway is started when C3 is hydrolyzed on a cell membrane of microorganisms. In hydrolyzed C3, complement B factors (CFBs) are bound to form a complex. When a complement D factor (CFD) operates on this complex, a function as the C3 convertase is obtained; thus, C3 is decomposed into C3a and C3b. A lectin pathway is started when mannose binding lectin (MBL), which is one kind of serum lectin, or the like is bound with glycan such as mannose on a surface of a pathogen and activated. MASP, which is one kind of serine proteases, is activated and C2 and C4 are decomposed into C2a and C2b, and C4a and C4b, respectively. After that, similarly to the classical pathway, C4b and C2a form a complex. The C4b-C2a complex works as the C3 convertase and decomposes C3 into C3a and C3b. In this manner, each pathway converges to the activation of C3 in the complement system. Therefore, it is effective to inhibit the expression of C3 in order to suppress the excess activation or inappropriate regulation of the complement.

### <Complement component C3>

In this specification, "the complement component C3 (also called "C3" simply)" is one kind of proteins related to the complement system. However, C3 means to encompass all the synonymous words without particular limitations, and includes C3 existing in nature and mutants thereof. A biological species from which C3 is derived is not limited in particular and is preferably the same as an animal species of the cell to which the double-stranded RNA or the composition disclosed herein is supplied. For example, in the case of supplying the double-stranded RNA or the composition disclosed herein to a cell derived from a human, a base sequence based on a base sequence of human C3 may be used as a main sequence. Note that C3 derived from the human is described here as a preferred example; however, this technique is also applicable to C3 derived from the biological species including nonhuman mammalians and other animal species.

It is known that C3 is overexpressed in various illness and diseases. For example, it is suggested that the amount of expression of C3 is related to macular degeneration (retinal detachment, chorioretinal degeneration, retinal degeneration, photoreceptor degeneration, RPE degeneration, mucopolysaccharidosis, rod-cone dystrophy, cone-rod dystrophy, and cone degeneration), age-related macula, cancer, Stargardt disease, Best disease, paroxysmal nocturnal hematuria, atypical hemolytic uremic syndrome, rheumatoid arthritis, neurodegenerative diseases, and the like. Moreover, the amount of expression of C3 is related to inflammatory responses and cell deaths along with these diseases and illness. Specifically, it is known that the expression of the protein in the complement system increases on the surface of the tumor cell. That is to say, the double-stranded RNA and the composition disclosed herein can suitably operate on the cells in which the amount of expression of C3 increases due to the aforementioned diseases, thereby being able to inhibit the growth thereof. In addition, in the activation of the complement system, the complement pathway converges to the activation of C3. Therefore, suppressing the expression of C3 is advantageous in that the growth of the cells can be suppressed surely. In addition, the inflammatory responses and anaphylaxis due to C3a can be suppressed.

### <Signal peptide region>

The base sequence of C3 is available from international databases. Examples of the international databases include National Center for Biotechnology Information (NCBI), European Nucleotide Archive (ENA), DNA Data Bank of Japan (DDBJ), UniPlot, Ensembl, and the like. Specifically, the base sequence of human C3 is provided in accession numbers NM_000064.4 and the like in NCBI. Note that the information about a signal peptide region of C3 can be obtained from the aforementioned international databases.

C3 includes about 1663 amino acid residues. The amino acid sequence set forth in SEQ ID No.: 5 includes 22 amino acid residues and shows the amino acid sequence of a signal peptide of human C3. The base sequence set forth in SEQ ID No.: 6 includes 66 bases and shows the base sequence of the signal peptide of human C3.

### <Double-stranded RNA>

The double-stranded RNA according to the present disclosure is the double-stranded RNA including the first strand and the second strand that is complementary to the first strand. Note that, here, the first strand is the sense strand and the second strand is the antisense strand in the following detailed description. The sense strand includes a main sequence including 19 bases to 23 bases in which a base at 5'-terminus is guanine (G) or cytosine (C), and an additional sequence including two bases to four bases added to 3'-terminus of the main sequence. In addition, the main sequence is determined from the base sequence including a part of the base sequence coding for the complement component C3, which is at least a part of the base sequence coding for the signal peptide region of the complement component C3.

The main sequence typically includes polynucleotides, which are polymers of ribonucleotides. In other words, the main sequence includes the RNA. That is to say, the base sequence of the main sequence is typically represented by the four letters of A (adenine), U (uracil), G (guanine), and C (cytosine) or the four letters of a, u, g, and c. However, in the attached sequence listing, uracil can be shown as thymine (T).

The main sequence of the sense strand can be the base sequence of a part of the base sequence coding for the signal peptide region of C3, for example. Thus, the double-stranded RNA can function as a small interfering RNA (siRNA) targeting C3. Moreover, since the base sequence of the signal peptide region of C3 exists on the upstream side of an mRNA, the expression of C3 can be effectively suppressed when the double-stranded RNA functions as the siRNA.

The double-stranded RNA disclosed herein can function as at least the siRNA. That is to say, it is predicted that such a double-stranded RNA will cause RNA interference (RNAi). The RNAi is a gene silencing process that suppresses the gene expression in a sequence-specific way by the short double-stranded RNA such as the siRNA. When the siRNA is transferred into the cell, the siRNA forms a complex called an RNA-induced silencing complex (RISC) with the protein in the cell. The RISC is bound with a homologous sequence of the mRNA transcribed from the gene to be targeted (here, C3 gene) and specifically cleaves the mRNA. Thus, the translation is inhibited.

The main sequence is selected from the base sequence coding for the signal peptide region of C3 preferably; however, one base or a plurality of bases (for example, two bases) may be substituted with another base, deleted, and/or added (inserted) in the range of achieving the effect of the present art.

When the entire main sequence is 100%, the ratio of the base sequence coding for the signal peptide region of C3 in the main sequence is, for example, preferably 45% or more, and may be 60% or more, 75% or more, 90% or more, or 100% or more.

The 5'-terminus of the main sequence is preferably guanine or cytosine. Since guanine and cytosine have a higher binding force to the complementary strand than adenine and uracil, the stability on the 5'-terminus of the sense strand (that is, on the 3'-terminus of the antisense strand) is enhanced. In other words, the stability on the 5'-terminus of the antisense strand becomes relatively low. Although the details of the mechanism are not clear, the RISC, which is the RNAi-related protein, tends to preferentially incorporate a chain of which the 5'-terminus is energetically less stable between the sense strand and the antisense strand. For this reason, when the 5'-terminus of the main sequence is guanine or cytosine, the antisense strand is easily incorporated into the RISC, and the RNAi can be more suitably induced. Thus, the double-stranded RNA can suitably function as the siRNA.

In five bases on the 3'-terminus of the main sequence, adenine and/or uracil is preferably contained by 60% or more (that is, three bases or more), may be contained by 80% or more (that is, four bases or more), or may be contained by 100% (that is, five bases). Accordingly, the stability on the 5'-terminus of the antisense strand becomes relatively lower than that on the 3'-terminus. As a result, the antisense strand can be easily incorporated into the RISC, and the RNAi can be more suitably induced.

The GC content (the total ratio of G and C in the entire base sequence constituting the main sequence) in the entire main sequence is not particularly limited, but may be, for example, 20% or more and 60% or less, is preferably 30% or more and 50% or less, or may be 30% or more and 45% or less. The GC content is a parameter related to, for example, the ease of cleavage of the RNA or a binding force between RNA having the main sequence and the antisense strand to be incorporated into the RISC. With the GC content described above, the effect of the RNAi can be efficiently exhibited.

For the main sequence, 19 to 23 bases can be selected from G or C of the gene coding for the signal peptide region of human C3. For example, the main sequence can be any one of the following base sequences:
GCCUGCUGCUCCUGCUACU (SEQ ID No.: 18);
CCUGCUGCUCCUGCUACUA (SEQ ID No.: 19);
CUGCUGCUCCUGCUACUAA (SEQ ID No.: 20); and
CUCUGGGGAGUCCCAUGUA (SEQ ID No.: 21).
Any of the base sequences set forth in SEQ ID Nos.: 18 to 21 includes the RNA. Any of the base sequences set forth in SEQ ID Nos.: 18 to 21 is specific to the C3 gene, making it possible to avoid the risk of inhibiting the translation of the mRNA of a host cell including a base sequence similar to a target sequence (so-called off-target effect). In addition, the double-stranded RNA including the base sequences set forth in SEQ ID Nos.: 18 to 21 as the main sequence, even in the low concentration, remarkably suppresses the growth of the abnormally growing cell; therefore, it is possible to avoid non-specific expression inhibition, non-specific cell growth inhibition, stress on the cell, or the like.

The base sequence set forth in SEQ ID No.: 1 (the DNA sequence corresponding to the RNA sequence set forth in SEQ ID No.: 18) is the 23rd to 41st base sequence of the base sequence coding for human C3 (that is, the sequence from a start codon to a stop codon). The base sequence set forth in SEQ ID No.: 2 (the DNA sequence corresponding to the RNA sequence set forth in SEQ ID No.: 19) is the 24th to 42nd base sequence of the base sequence coding for human C3. The base sequence set forth in SEQ ID No.: 3 (the DNA sequence corresponding to the RNA sequence set forth in SEQ ID No.: 20) is the 25th to 43rd base sequence of the base sequence coding for human C3. The base sequence set forth in SEQ ID No.: 4 (the DNA sequence corresponding to the RNA sequence set forth in SEQ ID No.: 21) is the 59th to 77th base sequence of the base sequence coding for human C3. Note that the base sequence set forth in SEQ ID Nos.: 1 to 3 is the base sequence of a part of the signal peptide region of human C3. The base sequence set forth in SEQ ID No.: 4 includes the base sequence of a part of the signal peptide region of human C3.

The double-stranded RNA including the main sequences set forth in SEQ ID Nos.: 18 to 21, by being supplied to at least one kind of cell, can suppress or inhibit the growth of that cell. Typically, when the double-stranded RNA is supplied to the tumor cell (for example, neuroblastoma, breast cancer, lung cancer, or the like), the growth of the tumor cell can be suppressed or inhibited. Note that C3 is expressed low in the normal cells other than the tumor cell but overexpressed in the tumor cell. Therefore, even if the double-stranded RNA disclosed herein is supplied to the normal cell, the amount of existence of C3 in the normal cell is relatively very small; therefore, it is considered that the double-stranded RNA hardly has an influence.

### <Additional sequence>

The sense strand of the double-stranded RNA disclosed herein can include the additional sequence including two bases to four bases added to the 5'-terminus or the 3'-terminus of the main sequence. The additional sequence is preferably added to the 3'-terminus of the main sequence. When the additional sequence is added, the RNAi can be more effectively induced.

The additional sequence includes a polynucleotide (dimer, trimer, or tetramer). The polynucleotide in the additional sequence may include only ribonucleotide, only deoxynucleotide, or both ribonucleotide and deoxynucleotide. That is to say, the sense strand and the antisense strand may be the RNA entirely or may be chimeric polynucleotide of RNA and DNA. In addition, the additional sequence may contain modified deoxyribonucleotide, modified ribonucleotide, another known nucleotide analog, or the like.

The base sequence included in the additional sequence preferably includes at least one base or more adenine, uracil, or thymine, although there is no particular limitation. In addition, from the viewpoint of improving the stability of the double-stranded RNA, the base sequence included in the additional sequence is more preferably thymine-thymine (TT).

### <Sense strand and antisense strand>

The sense strand includes, for example, the base sequence with 21 bases or more and 27 bases or less, and can include 21 bases or more and 25 bases or less, or 21 bases or more and 23 bases or less. In one preferred example, the sense strand includes 21 bases including 19 bases of the main sequence and 2 bases of the additional sequence. In the example, the RNAi can effectively be induced.

The antisense strand has the base sequence complementary to the main sequence of the sense strand. Accordingly, the antisense strand is hybridized with the sense strand to form a double-stranded structure. Alternatively, the base sequence of the antisense strand may be partially complementary to the main sequence of the sense strand. That is to say, one base or a plurality of bases (for example, two bases) of the antisense strand may be substituted with another base, deleted, and/or added (inserted). The function as the siRNA can be achieved as long as the sense strand and the antisense strand are capable of hybridization under at least the physiological condition. The complementary part of the base sequence typically includes a polymer of ribonucleotides (RNA).

In the double-stranded RNA according to the present disclosure, the sense strand or the antisense strand typically includes the ribonucleotide (RNA) that is not chemically modified. However, the double-stranded RNA according to the present disclosure may include DNA, chemically modified DNA or RNA, another known nucleotide analog, or the like to the extent that the art disclosed herein is not deteriorated remarkably. That is to say, one base or the plurality of bases (for example, two bases) of the sense strand or the antisense strand may be substituted with the RNA (or DNA) subjected to the chemical modification such as methylation or pseudouridylation. Examples of the chemically modified RNA include pseudouridine, N1-methyl pseudouridine, 5-methyl cytosine, inosine, and the like. For example, uridine of any one base or the plurality of bases (for example, two bases) in the double-stranded RNA according to the present disclosure can be substituted with pseudouridine.

In the double-stranded RNA according to the present disclosure, the antisense strand may include the main sequence complementary to the sense strand, and the additional sequence including two bases to four bases added to the 5'-terminus or the 3'-terminus of the complementary main sequence. The additional sequence may be added to the 3'-terminus of the complementary base sequence from the viewpoint of improving the function as the siRNA. In one preferred example, when the additional sequence of the sense strand is added to the 3'-terminus of the main sequence, the additional sequence of the antisense strand is added to the 3'-terminus of the complementary base sequence. The configuration of the additional sequence of the antisense strand may be similar to the configuration of the additional sequence of the sense strand described above. Typically, the base sequence of the additional sequence of the antisense strand is the same as that of the additional sequence of the sense strand to be hybridized, but may alternatively be a different base sequence.

The antisense strand includes, for example, the base sequence with 21 bases or more and 27 bases or less, and may include 21 bases or more and 25 bases or less, or 21 bases or more and 23 bases or less. The antisense strand includes the base sequence with the same length as the sense strand, and all or a part of the base sequence excluding the additional sequence includes the base sequence complementary to the main sequence of the sense strand. In one preferred example, the antisense strand includes the base sequence with the same length as the sense strand, and all the base sequence excluding the additional sequence includes the base sequence complementary to the main sequence of the sense strand.

### <Manufacturing method for double-stranded RNA>

The sense strand and the antisense strand included in the double-stranded RNA disclosed herein can be manufactured according to a general chemical synthesis method. For example, these can be synthesized using a commercially available DNA/RNA automatic synthesizer. Alternatively, the sense strand and the antisense strand may be synthesized in vitro or in vivo based on a genetic engineering technique. Note that the synthesized sense strand and antisense strand are preferably purified and can be purified using, for example, HPLC or the like.

The double-stranded RNA disclosed herein can be manufactured by, for example, annealing (hybridizing) the sense strand and the antisense strand. The annealing method may follow a conventionally known method; for example, annealing can be performed by mixing the sense strand and the antisense strand in equal amounts in a solvent, heating the mixture at 90°C for 1 to 5 minutes, and cooling the heated mixture to 4°C to room temperature. For example, distilled water, pure water, ultrapure water, a buffer (for example, HEPES-KOH buffer at pH 7.4, PBS, or the like), or the like can be used as the solvent. In order to prevent active RNase (RNA-degrading enzyme) from being mixed in the solvent, a solvent that has been subjected to, for example, a DEPC process, an autoclave process, and the like is preferably used.

### <Composition>

The composition disclosed herein includes the aforementioned double-stranded RNA. In addition to the aforementioned double-stranded RNA, the composition disclosed herein can include various carriers that are medically (pharmaceutically) acceptable depending on use modes. Regarding the carriers, for example, carriers commonly used as diluents, excipients, and the like in the medical fields are preferable. Such carriers vary as appropriate depending on the applications or modes of the composition. Typical examples thereof include water, a physiological buffer solution, various organic solvents, and the like. Moreover, the carrier can be an alcohol (such as ethanol) aqueous solution with a suitable concentration, glycerol, non-drying oil such as olive oil, liposome, or the like. As a secondary component that can be contained in a medical composition, various filling agents, bulking agents, binding agents, moisturizing agents, surfactants, dyes, flavoring agents, and the like are given. In addition, a carrier used in a conventionally known drug delivery system (DDS) can be included.

The mode of the composition disclosed herein is not particularly limited. For example, as the typical modes of the composition, modes of liquids, suspensions, emulsions, aerosols, foams, granules, powders, tablets, capsules, and ointments are given. Moreover, for the use in injection or the like, a lyophilized product or granulated product to prepare a drug solution by being dissolved in physiological saline or a suitable buffer (for example, PBS) or the like just before use can be used. The conventionally known methods may be used for the processes themselves to prepare various modes of medicines (compositions) from the double-stranded RNA (main component) and various carriers (secondary components) as materials, and a detailed explanation of such a manufacturing process itself is omitted herein because it is not a characterizing feature of the present disclosure. For example, Comprehensive Medicinal Chemistry, edited by Corwin Hansch, Pergamon Press, 1990, can be noted as a source of detailed information concerning formulations.

The composition disclosed herein inhibits the growth of at least one kind of cell. The cell whose growth is inhibited is the cell related to the expression of C3, and examples of such a cell include the tumor cell (for example, neuroblastoma, breast cancer cell, lung cancer, lymphoma, or the like), a liver cell, an eye cell, and the like. Among these cells, the composition according to the present disclosure inhibits the growth of the tumor cell suitably. That is to say, the double-stranded RNA and the composition according to the present disclosure can be suitably used as an antitumor drug (anticancer drug) that suppresses the growth of the tumor cell.

One aspect of the composition disclosed herein includes, in addition to the aforementioned double-stranded RNA, a peptide fragment with cell membrane permeability (cell penetrating peptide, CPP) that can transfer a foreign substance from outside the cell to the inside of a cytoplasm through a cell membrane. This peptide fragment configures a construct of the peptide fragment and the double-stranded RNA by directly or indirectly binding (linking) with the double-stranded RNA according to the present disclosure. In general, the double-stranded RNA has a negative charge and therefore cannot pass the cell membrane. However, for example, by directly or indirectly binding (linking) the double-stranded RNA according to the present disclosure to the N-terminus and/or the C-terminus of the peptide fragment, the construct of the peptide fragment and the double-stranded RNAdescribed above can be transferred to the inside of the cytoplasm. Note that the number of amino acid residues of the peptide fragment is not limited as long as the cell membrane permeability is not deteriorated.

In the case where the peptide fragment and the double-stranded RNA are bound to each other indirectly, for example, a linker is disposed between the peptide fragment and the double-stranded RNA. The kind of the linker is not limited in particular. The linker is typically a peptidic linker, a non-peptidic linker, or the like. A binding method for the peptide fragment and the double-stranded RNA is not limited in particular and can be implemented in accordance with various scientific methods that are conventionally known.

One aspect of the composition disclosed herein includes the peptide fragment and the double-stranded RNA according to the present disclosure. However, the double-stranded RNA does not need to be bound to the N-terminus or the C-terminus of the peptide fragment. In this aspect, for example, the double-stranded RNA and the peptide fragment can form a complex by an electric interaction or a molecular interaction. Such a complex can be transferred easily to the inside of a eukaryotic cell, making it possible to transfer the double-stranded RNA efficiently. The nucleic acid such as the double-stranded RNA is negatively charged, typically. Therefore, it is preferable that the peptide fragment to be used contain a high proportion of basic amino acid and be positively charged. Moreover, the ratio of the peptide fragment in this case may be 5 times to 100 times, and is preferably 40 times to 60 times that of the double-stranded RNA in terms of moles.

### <Manufacturing method for composition disclosed herein, and use thereof>

According to the present disclosure, a method of suppressing the growth of at least one kind of cell using the composition disclosed herein can be provided. The method disclosed herein includes a preparing step of preparing the composition according to the present disclosure, and a step of supplying the composition to a target cell.

In the preparing step, for example, the composition disclosed herein may be prepared by the conventionally known method as described above.

In the supplying step, the composition disclosed herein is supplied in vivo or in vitro to at least one kind of cell (for example, tumor cell or the like). The animal species of the cell to which the composition is supplied is not limited in particular and for example, mammals, birds, amphibians, reptiles, fishes, and the like may be given. The animal species from which C3 is derived and which serves as the foundation of the main sequence of the double-stranded RNA included in the composition and the animal species of the target cell are preferably the same. The kind of the target cell is not particularly limited either but is preferably the tumor cell and more preferably the neuroblastoma, the breast cancer, or the lung cancer. Note that the cell other than the tumor cell may exist at the destination to which the composition is supplied; however, the composition may be supplied to the target cell (that is, the tumor cell) only.

The method for administering the composition is not particularly limited and conventional methods used for treating animals may be used. The composition can be used in vivo in dosage and method according to the mode and intended purpose. For example, exactly a desired amount of liquid can be administered to a diseased area (for example, malignant tumor tissue, virus-infected tissue, inflamed tissue, etc.) of a patient or an animal individual (i.e., the body) by intravenous, intralymphatic, intramuscular, subdermal, intradermal, or intraperitoneal injection. Alternatively, the composition in a solid mode such as a tablet or in a gel-form or aqueous jelly-form such as ointment can be administered directly to a predetermined tissue (for example, a diseased area such as an organ or a tissue including a tumor cell, an inflamed cell, or the like). Further alternatively, the composition in a solid mode such as a tablet can be administered orally. In the case of the oral administration, it is preferable to perform encapsulation or apply a protection (coating) material in order to suppress decomposition by digestive enzymes in the alimentary canal.

The amount of the composition to be supplied in vivo is not limited in particular. For example, the lower limit value of the amount of the double-stranded RNA per kilogram of the animal individual can be 0.01 mg or more, 0.05 mg or more, or 0.1 mg or more. In addition, the upper limit

value of the amount of the double-stranded RNA per kilogram of the animal individual can be 10 mg or less, 5 mg or less, or 1 mg or less, for example. Moreover, the amount of the composition to be supplied in vitro is not limited in particular. In a culture solution of a supply target object such as a cell, the lower limit value of the concentration of the double-stranded RNA can be, for example, 1 nM or more, 5 nM or more, or 10 nM or more. The upper limit value of the concentration of the double-stranded RNA in this culture solution can be, for example, 10 µM or less, 5 µM or less, 2 µM or less, 1 µM or less, or 100 nM or less.

The composition disclosed herein can be supplied to the inside of the target cell by a known transfection method. Examples of such a method can include a chemical gene transfer method using a cationic molecule (commercial transfection reagent or the like), a physical transfer method such as micro-injection or electroporation, a biological gene transfer method using virus, and the like. Moreover, the composition may be supplied to the inside of the cell using the peptide fragment with the cell membrane permeability as described above.

### [Examples]

Several test examples concerning the art disclosed herein are described below, but it is not intended to limit the art disclosed herein to these test examples.

### <Preparation of double-stranded RNA>

The polynucleotides with the base sequences set forth in SEQ ID Nos.: 7 to 21 were synthesized artificially. The base sequences of the respective polynucleotides are shown in Table 1. In each polynucleotide, "TT" (additional sequence) on the 3'-terminus is the DNA, and the other part of the sequence (main sequence) includes the RNA. The obtained polynucleotides were used to prepare the double-stranded RNA used in each of Samples 1 to 5 shown in Table 1 by annealing the sense strand and the antisense strand having the complementary sequences. The double-stranded RNA used in each of Samples 1 to 5 was dissolved in PBS so that the concentration of the RNA became 2 mM and thus, an RNA solution was prepared.

### [Table 1]

**Table 1**

| Structure of double-stranded RNA | | | |
|---|---|---|---|
| Sample 1 | Sense strand: | 5' GCCUGCUGCUCCUGCUACUTT 3' | (SEQ ID No.: 8) |
| | Antisense strand: | 3' TTCGGACGACGAGGACGAUGA 5' | (SEQ ID No.: 9) |
| Sample 2 | Sense strand: | 5' CCUGCUGCUCCUGCUACUATT3' | (SEQ ID No.: 10) |
| | Antisense strand: | 3' TTGGACGACGAGGACGAUGAU 5' | (SEQ ID No.: 11) |
| Sample 3 | Sense strand: | 5' CUGCUGCUCCUGCUACUAATT3' | (SEQ ID No.: 12) |
| | Antisense strand: | 3' TTGACGACGAGGACGAUGAUU 5' | (SEQ ID No.: 13) |
| Sample 4 | Sense strand: | 5' CUCUGGGGAGUCCCAUGUATT3' | (SEQ ID No.: 14) |
| | Antisense strand: | 3' TTGAGACCCCUCAGGGUACAU 5' | (SEQ ID No.: 15) |
| Sample 5 | Sense strand: | 5' UUUCUAGCAAAGAUUUUUUTT3' | (SEQ ID No.: 16) |
| | Antisense strand: | 3' TTAAAGAUCGUUUCUAAAAAA 5' | (SEQ ID No.: 17) |

As shown in Table 1, the sense strand of the double-stranded RNA according to Sample 1 includes the main sequence set forth in SEQ ID No.: 18 (a part of the base sequence coding for the signal peptide region of C3) and the additional sequence including TT added to the 3'-terminus of the main sequence. The sense strand of the double-stranded RNA according to each of Samples 2 to 4 shown in Table 1 similarly includes the main sequence set forth in SEQ ID Nos.: 19 to 21 (the sequence including the base sequence coding for the signal peptide region of C3) and the additional sequence including TT added to the 3'-terminus of the main sequence. The sense strand of the double-stranded RNA according to Sample 5 includes the main sequence set forth in SEQ ID No.: 7 (the sequence manufactured artificially at random) and the additional sequence including TT added to the 3'-terminus of the main sequence. The antisense strand in each Example includes the sequence complementary to the main sequence and the additional sequence including TT added to the 3'-terminus of that sequence.

### <Cell growth test of human neuroblastoma cells>

As the tumor cell, an SK-N-SH line, which is a human neuroblastoma cell, was used. The SK-N-SH cell was precultured in 10% fetal bovine serum (FBS) + E-MEM (manufactured by FUJIFILM Wako Pure Chemical Corporation, Cat No. 051-07615) + 1% MEM non-essential amino acid solution (manufactured by FUJIFILM Wako Pure Chemical Corporation, Cat No. 139-15651), which was a culture medium. Note that 0.5% penicillin-streptomycin (manufactured by FUJIFILM Wako Pure Chemical Corporation, Cat No. 168-23191) was added to the culture medium only at the preculture; however, the penicillin-streptomycin was not added at the following culture and evaluation.

The SK-N-SH cell that adhered to a culture plate on the first day was cleaned with PBS and then, a 0.25%-trypsin/EDTA solution was added thereto, and incubation was performed for two minutes at 37°C. After the incubation, the culture medium described above was added to deactivate trypsin. After that, five-minute centrifugal separation was performed at 150 × g to precipitate the cells. After the supernatant generated by the centrifugal separation was removed, the culture medium described above was added to the precipitate (cell pellet) to prepare a cell suspension of about 5 × 10⁴ cells/mL. One commercial 96-well plate was prepared and the cell suspension was seeded in each well so that 5 × 10³ cells/100 µL/well was satisfied, and the incubation was carried out for one night at 37°C under 5% CO₂.

### (Sample 1)

On the second day, 75 µL of Opti-MEM (registered trademark) and 3 µL of the RNA solution prepared to be 2 mM with PBS were mixed; thus, a solution A was prepared. Moreover, 4.5 µL of Lipofectamine (registered trademark) RNAiMAX and 75 µL of Opti-MEM (registered trademark) were mixed; thus, a solution B was prepared. Next, the solution A and the solution B were mixed in equal amounts to prepare a solution C, which was incubated at room temperature for five minutes. The prepared solution C was added to the well where the SK-N-SH cell was cultured so that 11 µL/well was satisfied (the final concentration of the double-stranded RNA became 4 µM). After that, the incubation was carried out for three days at 37°C under 5% CO₂.

The growth of the cell was evaluated using Cell Counting Kit-8 (CCK-8, DOJINDO LABORATORIES). On the fifth day (three days after the siRNA was added), the 96-well plate where the SK-N-SH cell was cultured was extracted, 10 µL of CCK-8 was added to each well, and the incubation was carried out for 1.5 hours at 37°C under 5% CO₂. The light absorbance at 450 nm in each well was measured. Note that the light absorbance was the average value of the three wells. As the blank, a well including only the culture medium and the CCK-8 reagent was provided. The value obtained by subtracting the light absorbance of the blank from the light absorbance in Sample 1 was used as the measurement value in Sample 1.

### (Samples 2 to 5)

Samples 2 to 5 were obtained in a manner similar to Sample 1 except that the double-stranded RNA according to Sample 1 was changed to a double-stranded RNA according to each of Samples 2 to 5 shown in Table 1.

### (Comparative Example)

Comparative Example was performed in a manner similar to Sample 1 except that a PBS solution was used instead of the RNA solution in Sample 1. That is to say, the double-stranded RNA was not transferred in Comparative Example.

An unprocessed well was prepared in a manner similar to Sample 1 except that the RNA solution and Lipofectamine (registered trademark) RNAiMAX were not added. The cell viability in each test example, which is represented by the ratio when the measurement value of the unprocessed well is 100%, is shown in Fig. 2. Fig. 2 shows the results of the test using the double-stranded RNA shown in Table 1, that is, the results of the double-stranded RNA in which the sequence (SEQ ID Nos.: 1 to 4) including the base sequence coding for the signal peptide region of C3 is the main sequence with respect to the neuroblastoma cell.

As shown in Fig. 2, the cell viability in Samples 1 to 4 was low and was much lower than in Comparative Example. In addition, the cell viability was the lowest in Sample 2 (SEQ ID No.: 2). The aforementioned test results indicate that the double-stranded RNA in each of Samples 1 to 4 has a function of inhibiting the growth of the tumor cell (neuroblastoma cell). In addition, the base sequence set forth in SEQ ID No.: 7 is a random sequence containing substantially the same amount of ATCG as the base sequences set forth in SEQ ID Nos.: 1 to 4. Sample 5 in which SEQ ID No.: 7 described above was the main sequence did not have an influence on the growth of the cell. Therefore, the sequences of these SEQ ID Nos.: 1 to 4 are specific to the C3 gene. Accordingly, the double-stranded RNA according to each of Samples 1 to 4 can avoid the off-target effect and does not have an influence on other organs; thus, the clinical applications thereof can be much expected.

### <Cell growth test of human neuroblastoma cell using double-stranded RNA with low concentration>

The double-stranded RNA used in each of Samples 1 to 4 shown in Table 1 was prepared. The double-stranded RNA shown in each of Samples 1 to 4 was dissolved in PBS so that the concentration of each RNA became 2 mM; thus, the RNA solution was prepared. After that, the solution was diluted further by 10 times with PBS; thus, a low-concentration RNA solution in which the concentration of the RNA was 200 µM was prepared. Tests were conducted similarly to the cell growth test for the human neuroblastoma cell except that the aforementioned low-concentration RNA solution was used. That is to say, the final concentration of the double-stranded RNA added to the well where the SK-N-SH cell was cultured was set to 0.4 µM. Note that the cell viability in each test example was expressed by the ratio when the measurement value of the unprocessed well was 100%.

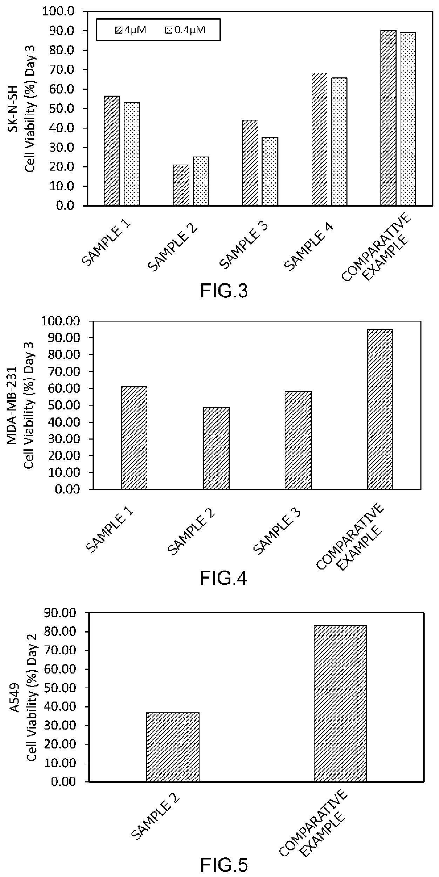

Fig. 3 is a graph in which the cell viability was compared between when the final concentration of the added double-stranded RNA was set to 4.0 µM and 0.4 µM. As shown in Fig. 3, the cell viability of Samples 1 to 4 is low. Moreover, the cell viability of Samples 1 to 4 was much lower than that of Comparative Example. This indicates that the double-stranded RNA of each of Samples 1 to 4 had a function of inhibiting the growth of the tumor cell (human neuroblastoma cell) even at the low concentration. In addition, the double-stranded RNA of each of Samples 1 to 4 had the equal or greater cell inhibiting function even if the concentration was 1/10. Therefore, since the double-stranded RNA of each of Samples 1 to 4 has the sufficient growth inhibiting function for the tumor cell even at the low concentration, the non-specific expression inhibition or the non-specific cell growth inhibition can be avoided and the clinical applications can be expected sufficiently.

### <Cell growth test for human breast cancer cell>

The cell growth test was performed similarly to the cell growth test for the human neuroblastoma cell except that an MDA-MB-231 line, which is a human breast cancer cell, was used as the tumor cell. The cell viability in each test example is expressed by the ratio when the measurement value of the unprocessed well is 100% and is shown in Fig. 4.

As shown in Fig. 4, the cell viability of Samples 1 to 4 was low and much lower than that of Comparative Example. This indicates that the double-stranded RNA of each of Samples 1 to 4 has a function of inhibiting the growth of the tumor cell (breast cancer cell).

### <Cell growth test of A549 line>

A549 line, which is a human lung cancer cell, was used as the tumor cell. In addition, the growth of the cell was evaluated in such a way that the 96-well plate where the A549 cell was cultured was extracted on the fourth day (two days after siRNA was added), 10 µL of CCK-8 was added to each well, and the incubation was carried out for 2.0 hours at 37°C under 5% CO₂. Except this, the test was performed similarly to the cell growth test of the human neuroblastoma cell. The cell viability in each test example, which is represented by the ratio when the measurement value of the unprocessed well is 100%, is shown in Fig. 5.

As shown in Fig. 5, the cell viability of Sample 2 was low and much lower than that of Comparative Example. This indicates that the double-stranded RNA of Sample 2 has a function of inhibiting the growth of the tumor cell (lung cancer cell).

The specific examples of the art disclosed herein have been shown above in detail; however, these are just examples and will not limit the scope of claims. The art described in the scope of claims includes those in which the specific examples given above are variously modified and changed.

In the art disclosed herein, the components and the processes herein described can be omitted or combined as appropriate unless a particular problem will occur. In addition, this specification includes the disclosure described in the following items.
Item 1: The double-stranded RNA including the first strand and the second strand that is complementary to the first strand, in which the first strand includes the main sequence including 19 or more and 23 or less bases, in which the base at the 5'-terminus is guanine (G) or cytosine (C), and the additional sequence including the two or more and four or less bases added to the 3'-terminus of the main sequence, and the main sequence includes a part of the base sequence coding for the complement component C3, which is at least a part of the base sequence coding for the signal peptide region of the complement component C3.
Item 2: The double-stranded RNA according to Item 1, in which the second strand includes the main sequence complementary to the first strand, and the additional sequence including the two to four bases added to the 3'-terminus of the complementary main sequence.
Item 3: The double-stranded RNA according to Item 1 or 2, in which at least the three bases among the five bases on the 3'-terminus of the main sequence are adenine (A) and/or uracil (U).
Item 4: The double-stranded RNA according to any one of Items 1 to 3, in which the base sequence coding for the signal peptide region of the complement component C3 includes any of the following base sequences:
   GCCTGCTGCTCCTGCTACT (SEQ ID No.: 1);
   CCTGCTGCTCCTGCTACTA(SEQ ID No.: 2);
   CTGCTGCTCCTGCTACTAA (SEQ ID No.: 3); and
   CTCTGGGGAGTCCCATGTA (SEQ ID No.: 4).
Item 5: The double-stranded RNA according to any one of Items 1 to 4, in which the base sequence included in the additional sequence is thymine-thymine (TT).
Item 6: The composition including the double-stranded RNA according to any one of Items 1 to 5, the composition inhibiting the growth of at least one kind of cell.
Item 7: The composition according to Item 6, in which the cell is the tumor cell.
Item 8: The composition according to Item 6 or 7, further including the peptide fragment with the cell membrane permeability that can transfer the foreign substance from outside the cell to the inside of the cytoplasm through the cell membrane.
Item 9: The method of suppressing the growth of at least one kind of cell, including:
   the preparing step of preparing the composition according to any one of Items 6 to 8; and
   the step of supplying the composition in vitro or in vivo to the cell.
Item 10: The method according to Item 9, in which the biological species of the cell is the same as the biological species including the complement component C3.

### [Industrial Applicability]

The double-stranded RNA disclosed herein can inhibit (or suppress) the growth of the cell as described above. Therefore, using the double-stranded RNA can provide the composition (for example, antitumor drug) that inhibits the growth of at least one kind of cell (for example, the tumor cell).

## Claims

1. A double-stranded RNA comprising a first strand and a second strand that is complementary to the first strand, wherein
the first strand includes a main sequence including 19 or more and 23 or less bases, in which a base at a 5'-terminus is guanine (G) or cytosine (C), and an additional sequence including two or more and four or less bases added to a 3'-terminus of the main sequence, and
the main sequence includes a part of a base sequence coding for a complement component C3, which is at least a part of a base sequence coding for a signal peptide region of the complement component C3.

2. The double-stranded RNA according to claim 1, wherein the second strand includes a main sequence complementary to the first strand, and an additional sequence including two or more and four or less bases added to a 3'-terminus of the complementary main sequence.

3. The double-stranded RNA according to claim 1, wherein at least three bases among five bases on the 3'-terminus of the main sequence are adenine (A) and/or uracil (U).

4. The double-stranded RNA according to claim 1, wherein the base sequence coding for the complement component C3 includes any of the following base sequences:
GCCTGCTGCTCCTGCTACT (SEQ ID No.: 1);
CCTGCTGCTCCTGCTACTA(SEQ ID No.: 2);
CTGCTGCTCCTGCTACTAA (SEQ ID No.: 3); and
CTCTGGGGAGTCCCATGTA (SEQ ID No.: 4).

5. The double-stranded RNA according to claim 1, wherein a base sequence included in the additional sequence is thymine-thymine (TT).

6. A composition comprising the double-stranded RNA according to any one of claims 1 to 5, the composition inhibiting growth of at least one kind of cell.

7. The composition according to claim 6, wherein the cell is a tumor cell.

8. The composition according to claim 7, further comprising a peptide fragment with cell membrane permeability that can transfer a foreign substance from outside the cell to an inside of a cytoplasm through a cell membrane.

9. A method of suppressing growth of at least one kind of cell, comprising:
a preparing step of preparing the composition according to claim 6; and
a step of supplying the composition in vitro to the cell.

10. The method according to claim 9, wherein a biological species of the cell is the same as a biological species including the complement component C3.
